Europäisches Patentamt

European Patent Office

Office européen des brevets

(11) Publication number: **0 183 469**
**A1**

(12) **EUROPEAN PATENT APPLICATION**

(21) Application number: **85308408.5**

(22) Date of filing: **19.11.85**

(51) Int. Cl.⁴: **C 12 N 15/00**
**C 12 N 1/20**
**//C12R1:19**

(30) Priority: **21.11.84 US 673955**

(43) Date of publication of application:
**04.06.86 Bulletin 86/23**

(84) Designated Contracting States:
**AT BE CH DE FR GB IT LI LU NL SE**

(71) Applicant: **GENENTECH, INC.**
**460 Point San Bruno Boulevard**
**South San Francisco California 94080(US)**

(72) Inventor: **Lin, Norm Shin Chsiang**
**698 Crane Avenue**
**Foster City California 94404(US)**

(72) Inventor: **Palmer, Donna Thourson**
**24 East Park Street**
**Westerville Ohio 43081(US)**

(72) Inventor: **Miller, Harvey Ivan**
**207 Powell Avenue**
**Pleasant Hill California 94523(US)**

(74) Representative: **Armitage, Ian Michael et al,**
**MEWBURN ELLIS & CO. 2/3 Cursitor Street**
**London EC4A 1BQ(GB)**

(54) Phage resistant E. Coli bacterium and method for making the same.

(57) Bacteriophage resistant *E. coli* and methods for making the same where such bacteria have an essentially irreversible resistance to infection by certain phage due to the transposon induced deletion or inversion of a DNA sequence associated with a bacterial gene required for certain phage infection.

./...

E.coli. W3110

① TRANSDUCE WITH $\lambda$ :: Tn10

E.coli. W3110 :: Tn10

② SELECT FOR RESISTANCE TO $\lambda\Phi80$ INFECTION

E.coli. W3110 :: Tn10 - $\lambda\Phi80^R$

P1 LYSATE

③ — E.coli. AT982

TRANSDUCE AT982

E.coli. AT982 :: Tn10 Dap$^+$

P1 LYSATE

④ — E.coli. W3110

SELECT FOR RESISTANCE TO $\lambda\Phi80$ INFECTION AND TETRACYLINE RESISTANCE

E.coli. W3110 fhuA :: Tn10 - $\lambda\Phi80$

⑤ SELECT FOR REVERSION TO TETRACYLINE SENSITIVITY AND RESISTANCE TO $\lambda\Phi80$ INFECTION

E.coli. W3110 fhuA$^-$

1

PHAGE RESISTANT E. COLI BACTERIUM
AND METHOD FOR MAKING THE SAME

The present invention relates to bacteria which have an essentially irreversible resistance to certain phage infection. Moreover, the invention relates to methods for making such phage resistant bacteria.

Bacterial cultures are susceptible to infection by a wide variety of bacteriophages. Certain phage may infect a given bacterial culture resulting in the destructive lysis of the culture. In the past, such phage infection could be eliminated by destroying the phage infected bacterial culture. These bacterial cultures were typically small scale cultures which, if destroyed, could be readily replenished from non-infected sources. Recent technological advances in the field of genetic engineering have created the potential to produce relatively large quantities of biologically active recombinant polypeptides. Mass production of such biological products, however, typically requires the fermentation of large quantities of genetically engineered bacteria. The effort and cost to generate such large scale cultures has created a need to protect them from destructive phage infection.

Escherichia coli (E. coli) is a common host used to express recombinant polypeptides. One of the most virulent bacteriophages which may infect and lyse certain strains of E. coli is bacteriophage T1 (hereinafter T1). Infection by T1 is difficult to control since T1 is relatively air stable. Whereas in the past T1 infection could be overcome by periodically eliminating all cultures from a facility, in large fermentation facilities such culture elimination may require the destruction of large quantities of bacterial culture. Aside from the time and cost of regenerating such genetically engineered cultures from uncontaminated stock, such contamination may also destroy the parental strain for such genetically engineered cultures.

Bacteriophage T1 and bacteriophage φ80 (hereinafter φ80) are known to require at least two membrane proteins to infect wild-type E. coli (1). One of these proteins, fhuB (also known as tonB), is required for a number of iron transport systems (2). Another membrane protein is fhuA (also known as tonA). This outer membrane protein is required for the translocation of ferrichrome across the outer membrane and is used by T1 and φ80 to initiate bacterial infection by adsorption to this receptor (5). Bacteriophage T5 (hereinafter T5) also must interact with at least fhuA to infect (2). It does not, however, require the presence of fhuB protein (2). The genes which encode for these proteins are also designated fhuA and fhuB.

A mutant E. coli bacterium which is deficient in the production of functional fhuA and/or fhuB protein would be expected to be resistant to T1 as well as φ80. Such deficiency in the production of functional membrane proteins may be caused by point mutations. For example, a point mutation may convert a codon for an amino acid in the wild-type sequence, to one of three known termination codons. Such a mutation, early in the protein sequence, could produce a small non-functional polypeptide. Similarly, a point mutation could result in the conversion of a wild-type codon to one coding for a different amino acid. The resulting amino acid substitution, if in a critical part of the protein, could render the entire molecule non-functional. Point mutations, however, may revert spontaneously, thereby regenerating phage susceptibility.

Deletions of chromosomal material may also disrupt gene function. Deletions may be point deletions, i.e., deletion of a single nucleotide or sequence deletions which remove more than one nucleotide. A deletion within a particular gene generally results in an irreversible mutation. It has been postulated that two φ80 and T5 resistant strains of E. coli, derived as either spontaneous or chemically induced mutants, are the result of a deletion covering part of the fhuA gene and a previously unknown fhuC gene (9). These mutants, however, have difficulty utilizing rhodotorulic acid and possibly other hydroxamate siderophores which facilitate the transport of ferric ions across the cell membrane (9).

Besides point mutations and deletions, the DNA sequence encoding a membrane protein may be altered by the insertion of a transposon into the gene. Transposons are naturally occurring DNA sequences which may insert into a plurality of sites within a bacterial genome. Many transposons carry genes which confer resistance to a variety of antibiotics, e.g., tetracycline, streptomycin, kanamycin, etc. (11). This antibiotic resistance provides a characteristic which may be used to select bacterial strains which incorporate a given transposon into a genome. Transposon Tn10, which carries tetracycline resistance, has been used to isolate mutants of E. coli K-12 which resist T1 infection (9). These mutants have been characterized as having a transposon insertion which disruptes the normal expression of fhuA or fhuB. These mutants may, however, revert to T1 susceptibility by the spontaneous precise excision of the transposon coupled with the loss of tetracycline resistance.

The need, therefore, exists for a bacterium which is resistant to T1 infection, which does not revert to phage susceptibility and which substantially maintains wild-type growth characteristics.

Accordingly, it is desirable in particular to provide an E. coli bacterium which has a chromosomal deletion or inversion of a DNA sequence that confers essentially irreversible resistance to T1 phage infection and methods for making the same.

It is further desirable in particular to provide an E. coli bacterium which has a deletion or inversion of a DNA sequence associated with the fhuA gene, thereby rendering the bacterium resistant to infection by the phages T1, T5 and $\phi$80 and methods for making the same.

According to one aspect of the present invention there is provided a mutant E. coli bacterium derived from a wild-type E. coli bacterium, said mutant bacterium being essentially irreversibly resistant to phage which infect said wild-type bacterium by use of at least one membrane protein in said wild-type bacterium, said mutant being characterized by a transposon generated deletion or inversion of DNA sequence associated with at least one of said wild-type membrane proteins.

According to another aspect of the present invention there is provided a method for making a mutant E. coli bacterium derived from a wild-type E. coli bacterium, said mutant bacterium being essentially irreversibly resistant to phage which infect said wild-type bacterium by adsorption to at least one membrane protein in said wild-type bacterium comprising:

selecting a first bacterial strain from an E. coli transposon hop pool, wherein said first bacterial strain contains said transposon and is resistant to said phage, and

selecting from said first bacterial strain at least one modified first bacterial strain characterised by the loss of a selection characteristic of said transposon and by a transposon generated deletion or inversion of a DNA sequence associated with at least one of said wild-type E. coli membrane proteins, thereby rendering said selected modified first bacterial strain essentially irreversibly resistant to said phage.

In general, the present invention is directed to bacteria which have an essentially irreversible resistance to infection by a particular phage or group of phages. The resistance is imparted by generating a bacterium with a deletion or inversion of a DNA sequence associated with a gene or genes which are required by a particular phage or group of phages to infect the bacteria. The present invention is also directed to methods for making such phage resistant bacteria.

6

a gene or genes which are required by a particular phage or group of phages to infect the bacteria. The present invention is also directed to methods for making such phage resistant bacteria.

According to the present invention, a bacterium with an essentially irreversible resistance to T1 may be made by generating a deletion or inversion of a DNA sequence associated with a gene required for T1 infection. A transposon which carries antibiotic resistance is first inserted into such a gene to generate a T1 phage resistant bacterium. The transposon is then allowed to imprecisely excise from the gene thereby producing either a deletion or inversion of a DNA sequence required for T1 infection.

More particularly, the present invention discloses methods for making an E. coli bacterium containing a deletion or inversion of a DNA sequence associated with the fhuA gene. Since fhuA is an outer membrane protein of E. coli which is required by the phages T1, T5 and φ80 to infect E. coli (2), the effect of a deletion or inversion of a fhuA DNA sequence is to impart in such bacteria an essentially irreversible resistance to infection by the phages T1, T5 and φ80.

Additional features of the invention will be evident from the following description taken in conjunction with the accompanying drawings wherein:

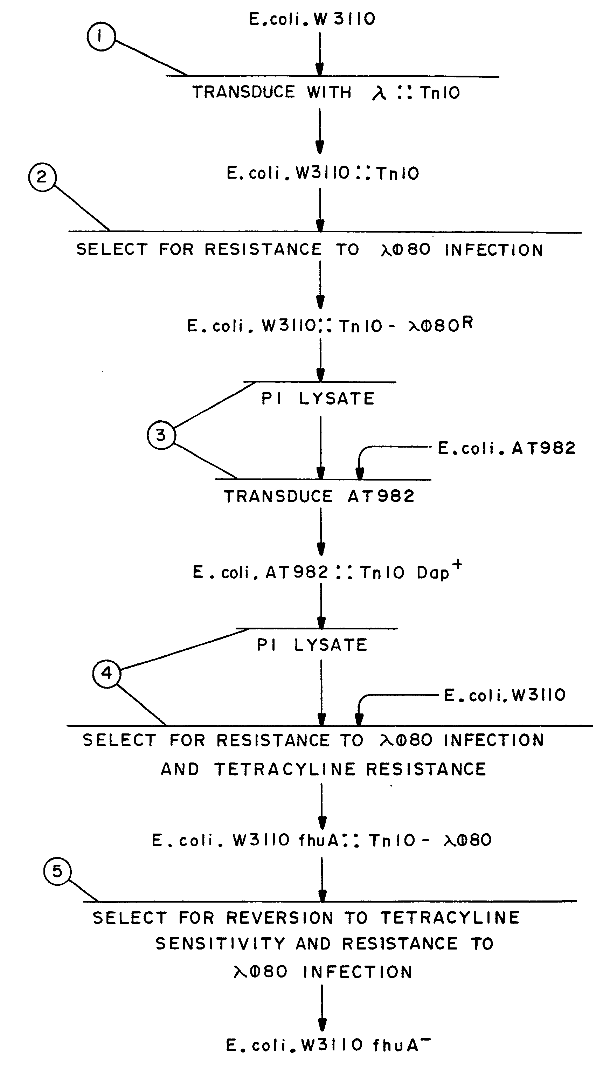

Figure 1 is a flow diagram of the method used in accordance with the present invention.

Resistance to the infection of E. coli bacteria by the bacteriophages T1 and φ80 may occur by mutations in the fhuA or fhuB genes of the E. coli chromosome. The fhuA and fhuB genes express membrane proteins that are both needed for infection by phages T1 and φ80 (2). Mutations in fhuB are pleiotrophic and may adversely effect the viability of such mutants. Mutations in the fhuA gene, however, are far less severe in their effects on the growth and reproduction of such fhuA mutants. Mutation in the fhuA gene is, therefore, preferable in generating a T1, T5 and φ80 resistant mutant capable of propagation and overall gene expression at rates equal to or not substantially less than the non-mutated, wild-type E. coli bacterium.

Mutations in the fhuA gene may be characterized as point mutations, transposon insertions, deletions or inversions. Point mutations and transposon insertions may readily revert to regenerate the fhuA gene and T1 susceptability. Deletions and inversions within the fhuA gene are essentially irreversible events and are therefore preferred. Deletions may occur spontaneously or by chemical treatment (9). The isolation of a mutant with a spontaneous deletion in the fhuA gene in a bacterial strain used for expressing cloned genes would be fortuitous but highly improbable. Deletions caused by chemical mutagenesis, however, may result in multiple deletions within the genome which may adversely affect the viability of the mutant (9).

In the present invention, the imprecise excision of a transposon is used to generate a deletion or inversion of a DNA sequence associated with the fhuA gene. The effect of such a deletion or inversion is the loss of functional fhuA membrane protein as manifested by the complete absence of fhuA membrane protein or by a fhuA protein which is either incapable of binding to T1, T5

8

or ϕ80 or functionally incapable of binding to the cell membrane.

In general, a transposon generated deletion or inversion of a DNA sequence associated with the fhuA gene of E. coli comprises inserting into the fhuA gene a transposable element which contains an expressible selection characteristic, followed by the selection of strains which are resistant to T1, T5 or ϕ80 and which express the selection characteristic of the transposable element. The transposable element is then allowed to imprecisely excise from these strains to produce a strain lacking the selection characteristic of the transposable element but retaining the phage resistance due to deletion or inversion of all or part of a bacterial gene required for phage infection. Transposons with particular selection characteristics which may be used in practicing the present invention include: Tn10 - tetracycline resistance, Tn5 - kanamycin resistance, Tn3 - ampillicin resistance, Tn 9 - cloramphenicol resistance and other transposons known to those skilled in the art (11). In general, this method may also be used to generate deletions or inversions of DNA sequences within fhuA of fhuB or other wild-type genes required for phage infection.

The use of a transposon to generate deletions has several advantages. In general, the deletion occurs only in the vicinity of the transposon insertion (6). In addition, a variety of deletion mutants may be generated which have different amounts of a DNA deleted (6). A transposon generated deletion which primarily affects the fhuA gene may be isolated thereby limiting the potentially adverse effects of the deletion.

The use of the transposon to generate inversions is also advantageous in that such inversions generally occur between the transposon insertion site and a second locus on the genome (6). As such, a part of the gene containing the transposon may be inverted to the second locus and replaced by material from the second locus. This is accompanied by the loss of the transposon. In this manner the expression of the gene originally containing the transposon may be disrupted. Depending on where the second genomic locus is located, such an inversion may be mutagenic for a single gene at the second locus if the second locus is within a structural gene or produce a polar effect if the second locus is within an operon. If the second locus is not within a gene or operon, the effect of the inversion may only be the disruption of the gene which originally contained the transposon. Inversions which primarily affect genes required for phage infection may therefore be isolated.

A bacterium made according to the present invention and characterized by a transposon generated deletion or inversion of a DNA sequence associated with the fhuA or other wild-type gene required for phage infection may, therefore, be distinguished from naturally occurring mutants having deletions or inversions, in that, the present invention allows the selection of mutants which have essentially irreversible mutations which primarily affect the fhuA or other wild-type gene with little or no effect on other gene functions.

Naturally occurring mutants, on the other hand, may have deletions or inversions which may disrupt the functions of numerous genes. Such mutations typically result in a single mutated strain which precludes the selection of a strain with minimal disruption of other gene functions.

Further, chemically induced deletions may generate a number of strains with a variety of deletions within a bacterial genome. However, unlike transposon generated deletions, chemically induced deletions do not produce a range of deletions originating at one point on the bacterial genome. A mutant strain with minimal disruption of gene function may therefore be difficult to isolate. Therefore, mutant bacteria made according to the present invention would be expected to have deletions or inversions of a DNA sequence which are more limited to a specific gene than naturally occurring deletion or inversion mutants or chemically induced deletion mutants. A bacterium characterized by a transposon generated deletion or inversion of a DNA sequence associated with the fhuA or other wild-type gene is, therefore, a mutant bacterium with a deletion or inversion primarily affecting the fhuA or other wild-type gene.

In a preferred embodiment, E. coli strain W3110 is selected for constructing a W3110 bacterial strain containing a gene deletion or inversion of a DNA sequence associated with the fhuA gene. Strain W3110 is chosen because it is a common host strain for recombinant DNA product fermentations. This bacterial strain is the starting material for the method depicted as a flow chart in Figure 1.

In general, E. coli W3110 is transduced in step 1 of Figure 1 by a lambda bacteriophage containing the transposable element Tn10 (λ::Tn10). The transduction by bacteriophage λ::Tn10 results in the precise insertion of the Tn10 transposon at a plurality of sites within the bacterial genome without incorporating sequences from the bacteriophage. This bacterial pool is designated W3110::Tn10.

In step 2 in Figure 1, strains of W3110::Tn10 are selected for resistance to λɸ80 infection. Phage λɸ80 is chosen because it uses the fhuA protein to attach to E. coli and because λɸ80 is not as dangerous as T1 to E. coli stocks. These selected strains are designated W3110::Tn10-λɸ80R. Phage λɸ80 resistant strains of W3110::Tn10 are pooled and infected with bacteriophage P1. The resulting lysate is used to transduce E. coli AT982 (3) as depicted in step 3. Such transduction results in the recombination of DNA sequences of W3110::Tn10-λɸ80R DNA with homologous sequences present in AT982. Strain AT982 is chosen because it contains a dapD mutation. The dapD gene is known to be located close to the fhuA gene (1). As such, fhuA and dapD are 65 to 91% cotransducible (3). FhuB and dapD, however, are not cotransducible. Transduction of strain AT982 by a P1 lysate of W3110::Tn10-λɸ80R and selection of trans-ductants which are tetracycline resistant and which regenerate the dapD function indicates that such transductants may contain Tn10 within the fhuA gene.

Other dapD mutants of E. Coli may be used in step 2 (3), as well as E. coli strains containing mutations in other genes which are contransducible with the fhuA gene (1).

In step 4 of Figure 1, tetracycline resistant strains with regenerated dapD function are the source of DNA for the bacteriophage P1 transduction of E. coli W3110. As stated above, such transduction results in recombination at regions containing homologous sequences. Transduced W3110 strains expressing tetracycline resistance and λɸ80 resistance are selected. These strains are designated as E. coli. W3310 fhuA::Tn10-λϕ80R. Such strains are functionally equivalent to the W3110::Tn10-λϕ80R strain obtained from step 2. The W3110fhuA::Tn10-λϕ80R strains obtained from steps 3 and 4 are expected, however, to consist of strains with the Tn10 inserted in the fhuA gene rather than the fhuB gene or other unknown genes which may also result in resistance to T1, T5 or ϕ80 infection. Steps 3 and 4 therefore increase the likelihood of obtaining in step 5 a T1, T5 or ϕ80 phage resistant bacterium with a deletion or inversion of a DNA sequence associated with the fhuA gene. It is to be understood, however, that steps 3 and 4 are not required to practice the invention. They are included only to maximize the probability of isolating mutants with Tn10 in the fhuA gene and are not intended to limit the scope of the invention.

In step 5 of Figure 1, strains of E. coli W3110 fhuA::Tn10λϕ80R from step 4 or alternatively W3110::Tn10-λϕ80R from step 2 are selected on the basis of resistance to phage λϕ80 infection and reversion to tetracycline sensitivity (7). In reverting to tetracycline sensitivity, the Tn10 element may excise precisely or imprecisely. A precise excision may regenerate a functional fhuA gene. Imprecise excision, which occurs at a frequency of about $10^{-4}$ per bacterium, often results in a chromosomal deletion or inversion of sequences located close to the transposable element (6). Since strains of W3110fhuA::Tn10-λϕ80R have been selected in steps 3 and 4 to maximize the likelihood that Tn10 is located within the fhuA gene, imprecise excision of Tn10 from such strains may be expected to generate a deletion or inversion of the fhuA gene to produce the strains designated E. coli W3110 fhuA⁻.

## Construction of λ::Tn10

Lambda cI857 $b_{221}$ Oam 29 is the starting material for λ::Tn10 construction. This λ phage is characterized by the combination of three well known mutants of λ. The cI857 genotype produces a cl repressor which is reversibly temperature sensitive (12). The $b_{221}$ genotype (13, 17) is characterized by a deletion of non-essential λ DNA sequences. The $b_{221}$ genotype provides space within the λ genome to accomodate the Tn10 transposon. Further, the b 221 deletion removes the λ int (integration) gene thereby rendering such phage incapable of normal integration as a prophage in E. coli. The Oam 29 genotype is characterized by an amber mutation in the λ's O replication gene (14, 16, 18). This lambda cI857$b_{221}$ Oam29 is known to those skilled in the art (14) and is constructed from the above genotypes by standard procedures. The Tn10 derivative is also known to those skilled in the art and is constructed by the following procedures. A lysate of lambda cI857$b_{221}$Oam29, is prepared on the amber suppressor E. coli C600 (ATCC 23724) which has been manipulated by procedures known to these skilled in the art to also carry the Tn10 transposon (10). This lysate is used to infect E. coli C600 (λ cI857) which contains an amber suppressor and a lambda prophage carrying the cI857 genotype. The preferred multiplicity of infection is 10. After incubation for one hour at 32°C, the cells are concentrated by centrifugutation and plated on broth plates containing 15 µg/ml tetracycline and 0.01M sodium pyrophosphate. The plates are incubated overnight at 32°C. Lysates of tetracycline resistant colonies are prepared by heat induction. The tetracycline resistant colonies are first grown in broth at 32°C after which the temperature is raised to 42°C for 90 minutes. This lysate is plated on E. coli C600. The plaques appearing on E. coli C600 are replica plated at 32°C onto E. coli C600 and E. coli W3102 sup+(λimm434) which contains the heteroimmune prophage lambda imm434 (16). Plaques appearing on the heteroimmune strain are plated onto tetracycline plates The plaques that appear on these plates are capable of transducing tetracycline resistance and indicate that the phage contained in such plaques carry Tn10. The phage, so identified, are propagated in E. coli C600 and are designated λ::Tn10.

Other types of λ::Tn10 are known (16). As a consequence the λ::Tn10 used in conjunction with the present invention may consist of other λ derivatives which are as effective as the λ::Tn10 herein disclosed.

Construction of λ¢80

E. coli W3110 is infected with λ cI phage (15) and φ 80 phage (ATCC 11456a-B1). The multiplicity of infection for each phage is about 5. The cells are grown for 90 minutes at 37°C to produce a lysate. This lysate is plated on E. coli W3110 (φ80)/λ at 37°C. The plaques from this plate are subsequently purified on E. coli W3110. A lysate of the λ¢80 so produced is used to assay resistance to T1 infection.

Crosses between λ and φ80 have been known for some time (19). Other strains of λ and ¢80 may therefore also be used in practicing the present invention. Such recombinants, however, must contain the ¢80 tail region to bind the fhuA membrane protein.

0183469

Example 1:

E. coli W3110 is transduced with λ::Tn10 to generate a Tn10 hop pool of E. coli W3110. (10)

The E. coli W3110::Tn10 hop pool is grown in L broth at 37°C to a cell density of about 1 x 10$^9$/ml. 0.5 ml of the culture is centrifuged and the pellet is resuspended in 0.2 mls of a λ¢80 lysate containing 7.0 x 10$^9$ pfu. The phage is allowed to adsorb for 30 minutes at 37°C. The suspension is then spread on EMB plates supplemented with tetracycline (15 µg/ml). After an overnight incubation at 37°C, the light pink are pooled in 3 ml of L broth, grown overnight at 37°C, washed twice, and resuspended in L broth. A bacteriophage P1 kc lysate is made on this culture (8).

E. coli AT982 (no. 4546, E. coli Genetic Stock Center, New Haven, Conn.) is transduced to tetracycline resistance by this P1 kc lysate. Tranductants are selected on L broth plates supplemented with tetracycline (15 µg/ml) and (40 µg/ml) dap (diaminopimelic acid). The resulting transductants are screened for tetracycline resistance and the regeneration of the dap gene (dap$^+$, tet$^R$). dap$^+$, tet$^R$ transductants are then tested for λ¢80 resistance.

P1 kc lysates are then made on several dap$^+$, tet$^R$, λ¢80 resistant strains. The lysates are used to transduce E. coli W3110 to tetracycline resistance. The transductants are screened and selected for λ¢80 resistance.

Tetracycline sensitive isolates are selected from the W3110 fhuA::Tn10-λ¢80R transductants (7). These isolates are checked for λ¢80 resistance and tetracycline sensitivity after single colony purification.

DNA is isolated from several tetracycline sensitive λφ80 resistant mutants and digested with SstII. The SstII digested DNA is characterized by the Southern blot procedure using radioactively labelled and SstII digested λ::Tn10 DNA as a probe to determine if the Tn10 has excised (4). One of the tetracycline sensitive isolates is shown to have lost two of the Tn10 hybridization bands as compared to the hybridization between DNA from the λ::Tn10 and the parental W3110 fhuA::Tn10λφ80R. A third hybridization band has an altered mobility indicating that a deletion caused by the imprecise excision of Tn10 has occurred.

SDS-gel electrophoresis of outer membrane preparations from the strain with an imprecise Tn10 excision reveal that the band assumed to be the fhuA protein has an altered electrophoretic mobility as compared to the wild-type fhuA protein. The resulting protein is non-functional as a λφ80 phage receptor protein. A second independent strain which also has undergone imprecise excision of Tn10 shows no fhuA protein on the SDS gel.

Neither of these strains demonstrate reversion to tetracycline resistance or to λφ80 susceptability indicating that there is an imprecise excision of all or part of the Tn10 transposon together with either a partial or complete deletion of the fhuA gene.

It is apparent that the above described general method may be used to generate bacterial strains with deletions or inversions of a DNA sequence at sites other than the fhuA gene. Such deletions or inversions may impart desirable and permanent characteristics besides resistance to T1, T5 and φ80 phage infection. These bacteria and the methods herein disclosed to generate such bacteria are contemplated by the present invention.

Having described the preferred embodiment of the present invention, it will appear to those ordinarily skilled in the art that various modifications may be made to the disclosed embodiment, and that such modifications are intended to be within the scope of the present invention.

The references grouped in the following bibliography and respectively cited parenthetically by number in the foregoing text, are hereby incorporated by reference.

## BIBLIOGRAPHY

1.  Bachmann, B.J., and K.B. Low, 1980. Microbial. Reviews 44:1.

2.  Braun, V., and K. Hantke, 1981. In B.K. Ghosh (ed.) Organization of the Prokaryotic Cell Membranes. Vol. II, CRC Press, Inc., Florida. p. 21.

3.  Bukhari, A.I., and A.L. Taylor, 1971. J. Bacteriol. 105:844.

4.  Davis, R.W., D. Botstein, and J.R. Roth, 1980. Advanced Bacterial Genetics. Cold Spring Harbor Laboratory. N.Y.

5.  Hantke, K., and V. Braun, 1978. J. Bacteriol. 135:190.

6.  Kleckner, N., K. Reichart, and D. Botstein, 1979. J. Mol. Biol. 127:89.

7.  Naloy, S.R., W.D. Nunn, 1981. J. Bacteriol. 145:1110.

8.  Miller, J.H., 1972 Experiments in Molecular Biology. Cold Spring Harbor Laboratory. N.Y. p. 304.

9.  Kadner, R.J., et al., 1980. J. Bacteriol. 143:256.

-18-

bibliography
10. Kleckner, N. et al., 1977. J. Mol. Biol. 116:125

11. Kleckner, N., Tranposable Elements in Prokaryotes, Annual Review of Genetics 1981 Vol. 15 p. 342-404. Annual Reviews, Inc., Palo Alto CA.

12. Sussman R. and Jacob F., 1962. Compt. Rend. Acad.Sci. 254:1517.

13. Davidson N. and Szybalski, W. 1971. In The Bacteriophage Lambda (Hershey, A.D. ed), pp. 45-82, Cold Spring Harbor Laboratory, Cold Spring Harbor, N.Y.

14. Kleckner, N. 1977. J. Mol. Biol. 116:125.

15. Ptashne, M. 1971. In The Bacteriophage Lambda (Hershey A.D. ed.) pp. 221-237, Cold Spring Harbor Laboratory, Cold Spring Harbor, N.Y.

16. Kleckner, N., et al., 1978. Genetics 90:427-461

17. Court, D. and Oppenheim, A., 1983. In Lambda II (Hendrix, R. W. et al. ed.) pp 257-277, Cold Spring Harbor Laboratory, Cold Spring Harbor, N.Y.

18. Arber, W., et al., 1983. In Lambda II (Hendrix, R.W. et al. ed.) pp 433-466, Cold Spring Harbor Laboratory, Cold Spring Harbor, NY.

19. Singer, E. R., 1964. Virology 22:650-651

0183469

CLAIMS

1.     A mutant E. coli bacterium derived from a wild-type E. coli bacterium, said mutant bacterium being essentially irreversibly resistant to phage which infect said wild-type bacterium by use of at least one membrane protein in said wild-type bacterium, said mutant being characterized by a transposon generated deletion or inversion of DNA sequence associated with at least one of said wild-type membrane proteins.

2.     A mutant E. coli bacterium as in claim 1 wherein said mutant is characterized by said transposon generated deletion.

3.     A mutant E. coli bacterium as in claim 1 or claim 2 wherein a said DNA sequence is associated with the fhuA gene.

4.     A mutant E. coli bacterium as in any one of claims 1, 2 and 3 wherein said phage include a T1, T5 and φ80.

5.     A mutant E. coli bacterium as in any one of the preceding claims wherein said transposon is Tn10.

6.     A method for making a mutant E. coli bacterium derived from a wild-type E. coli bacterium, said mutant bacterium being essentially irreversibly resistant to phage which infect said wild-type bacterium by adsorption to at least one membrane protein in said wild-type bacterium comprising:
       selecting a first bacterial strain from an E. coli transposon hop pool, wherein said first bacterial strain contains said transposon and is resistant to said phage, and
       selecting from said first bacterial strain at least one modified first bacterial strain characterised by the loss of a selection characteristic of said transposon and by a transposon generated deletion or inversion of a DNA sequence associated with at least one of said wild-type E. coli membrane proteins, thereby rendering said selected modified first bacterial strain essentially irreversibly resistant to said phage.

7.    A method of claim 6 wherein said modified first bacterial strain is characterized by said transposon generated deletion.

8.    A method of claim 6 or claim 7 wherein said DNA sequence is associated with the fhuA gene.

9.    A method of any one of claims 6, 7 and 8 wherein said phage include T1, T5 and φ80.

10.    A method of any one of claims 6 to 9 wherein said transposon is Tn10 and said selection characteristic is tetracycline resistance.

11.    A bacterium made according to the method of any one of claims 6 to 10.

///

E.coli.W3110

① TRANSDUCE WITH $\lambda :: Tn10$

E.coli.W3110 $:: Tn10$

② SELECT FOR RESISTANCE TO $\lambda \Phi 80$ INFECTION

E.coli.W3110 $:: Tn10 - \lambda \Phi 80^R$

③ PI LYSATE

E.coli.AT982

TRANSDUCE AT982

E.coli.AT982 $:: Tn10$ Dap$^+$

④ PI LYSATE

E.coli.W3110

SELECT FOR RESISTANCE TO $\lambda \Phi 80$ INFECTION
AND TETRACYLINE RESISTANCE

E.coli.W3110 fhuA $:: Tn10 - \lambda \Phi 80$

⑤ SELECT FOR REVERSION TO TETRACYLINE
SENSITIVITY AND RESISTANCE TO
$\lambda \Phi 80$ INFECTION

E.coli.W3110 fhuA$^-$

| DOCUMENTS CONSIDERED TO BE RELEVANT | | | EP 85308408.5 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,A | JOURNAL OF BACTERIOLOGY, vol. 143, no. 1, July 1980 (Washington, DC) <br><br> R.J. KADNER et al. "Genetic Control of Hydroxamate-Mediated Iron Uptake in Escherichia coli" pages 256-264 <br><br> * Page 256 (abstract) * <br><br> ---- | 1-5 | C 12 N 15/00 <br><br> C 12 N 1/20 <br><br> // C 12 R 1:19 |

**TECHNICAL FIELDS SEARCHED (Int Cl 4)**

C 12 N

The present search report has been drawn up for all claims

| Place of search | Date of completion of the search | Examiner |
|---|---|---|
| VIENNA | 25-02-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82